# EUROPEAN PATENT SPECIFICATION

(11) **EP 3 299 016 B1**
(45) Date of publication and mention of the grant of the patent: **16.12.2020**
(21) Application number: 16795868.5
(22) Date of filing: 17.05.2016
(51) Int. Cl.: A61K 31/4015, A61P 25/08, A61K 9/00, A61K 9/08, A61K 9/20, A61K 9/48, A61K 47/26

(54) **USE OF DEXTROROTATORY OXIRACETAM IN PHARMACEUTICAL FIELD**
VERWENDUNG VON RECHTSDREHENDEM OXIRACETAM IM PHARMAZEUTISCHEN BEREICH
UTILISATION D'OXIRACÉTAM DEXTROGYRE DANS LE DOMAINE PHARMACEUTIQUE

(30) Priority: 18.05.2015 CN 201510253556
(43) Date of publication of application: 28.03.2018
(73) Proprietor: Chongqing Runze Pharmaceutical Co. Ltd., Chongqing 400042 (CN)
(72) Inventor: YE, Lei, Chongqing 400042 (CN)
(74) Representative: Kitzler, Michael
(86) International application number: PCT/CN2016/082396
(87) International publication number: WO 2016/184381

(56) References cited:
- CN-A- 101 766 596
- CN-A- 101 766 596
- CN-A- 102 600 130
- CN-A- 102 600 130

## Description

### Technical field

The present invention relates to a use of dextrorotatory oxiracetam in pharmaceutical field.

### Description of Related Arts

Dextrorotatory oxiracetam ((R)-4-hydroxy-2-oxo-1-pyrrolidineacetamide) is the Dextrorotatory isomer of oxiracetam (Oxiracetam CAS 62613-82-5), CN102603607A discloses the preparation method. Compared with the racemic oxiracetam and S-oxiracetam, both of the racemic oxiracetam and S-oxiracetam can enhance memory, improve learning ability, improve the cognitive function of patients, and dextrorotatory oxiracetam is not effective in cognitive dysfunction. There is no report on the use of dextrorotatory oxiracetam alone as medicament application.

It is known in the industry that epilepsy is common in repeated attacks with loss of consciousness as well as loss of convulsions as the main symptoms of chronic neurological diseases, and its seizures are usually caused by a short-term abnormal synchronization activity of neurons in the brain; nevertheless its specific pathogenesis is still unclear. As a common disease, according to the World Health Organization statistics, about 50 million people around the world suffer from epilepsy. Epilepsy can occur at any time no warning before the general attack thereby resulting in great harm. Furthermore, epilepsy is often accompanied by depression, anxiety, migraine, infertility, low libido, autism and other complications, and therefore, treatment of epilepsy is very difficult.

At present, for antiepileptic drugs are phenytoin sodium, sodium valproate, carbamazepine, phenobarbital, lamotrigine, levetiracetam and so on. Clinical reactions show that these drugs have many side effects, mainly for mental development, language expression, learning and memory and other cognitive decline and craniofacial deformities, congenital heart defects, toe development and other fetal malformations. It has been reported that oxiracetam is used in the treatment of epilepsy patients for the treatment of cognitive and behavioral disorders after epilepsy. In essence, antiepileptic drugs play an inhibitory role in the central nervous system. Racemic oxiracetam enhance nerve excitement conduction, promote nervous system activity, enhance memory, improve learning ability, and improve forgetfulness. CN102600130 discloses oxiracetam or its optical isomers, preferably the levorotatory form of oxiracetam, for use in treating epilepsy. The development of new antiepileptic drugs is necessary.

### Summary of the Invention

The object of the present invention is to provide a dextrorotatory oxiracetam compound or a composition containing dextrorotatory oxiracetam compound for use in preventing or treating epilepsy.

The present invention is specifically related to a dextrorotatory oxiracetam compound or a composition containing dextrorotatory oxiracetam compound for use in preventing or treating acute epilepsy seizure.

The present invention is specifically related to a dextrorotatory oxiracetam compound or a composition containing dextrorotatory oxiracetam compound for use in preventing or treating generalized epilepsy seizure. For the above-mentioned uses, the dextrorotatory oxiracetam compound is prepared in a composition having dextrorotatory oxiracetam compound as an active ingredient. The dosage form of the drug may be an oral preparation such as a tablet, a dripping pill, a powder, a granule, a capsule, and the like; injections such as a powder for injection and a lyophilized powder for injection. The above dosage forms may be prepared according to a conventional method.

The above dosage forms are preferably oral capsules, tablets and injections.

The dosage of the above-mentioned dextrorotatory oxiracetam composition is 400 mg or more (including 400 mg), preferably 400 to 2000 mg/day, and more preferably 800 to 1600 mg/day.

The present invention relates particularly to a dextrorotatory oxiracetam compound or a composition containing dextrorotatory oxiracetam compound for use in preventing or treating epilepsy.

A composition for use in preventing or treating epilepsy contains dextrorotatory oxiracetam compound and a pharmaceutically acceptable excipient.

A composition for use in preventing or treating acute epilepsy seizure contains dextrorotatory oxiracetam compound and a pharmaceutically acceptable excipient.

A composition for use in preventing or treating generalized epilepsy seizure contains dextrorotatory oxiracetam compound and a pharmaceutically acceptable excipient.

The present invention is related to a dextrorotatory oxiracetam compound or a composition containing dextrorotatory oxiracetam compound for use in preventing or treating partial epilepsy seizure.

A composition for use in preventing or treating partial epilepsy seizure contains dextrorotatory oxiracetam compound and a pharmaceutically acceptable excipient.

The present invention is related to a dextrorotatory oxiracetam compound or a composition containing dextrorotatory oxiracetam compound for use in preventing or treating status epilepticus.

A composition for use in preventing or treating status epilepticus contains dextrorotatory oxiracetam compound and a pharmaceutically acceptable excipient.

In treating epilepsy comprising administering dextrorotatory oxiracetam to a patient, the mode of administration is preferably oral or injection.

The optical purity of the dextrorotatory oxiracetam compound is 99.0% or more, based on weight percent.
Stages: epileptic seizures evaluation are categorized by Racine Stages
Stage 0: no signs of any seizure;
Stage I: staring, head and face slightly trembling;
Stage II: head nodding or wet dog-like shaking;
Stage III: forelimb limit convulsions;
Stage IV: full-body ankylosing convulsions with a standing hind limb;
Stage V: full-body ankylosing clonic seizures with standing with fell;
Partial epilepsy seizure occurs when any one of Stage I to Stage III appears;
Generalized epilepsy seizure occurs when Stage IV or Stage V appears;
Status epilepticus (SE) occurs when Stage IV or Stage V lasts 30 minutes.

To further verifying the pharmaceutical effect of the present invention, the inventors have conducted the following tests. S-oxiracetam and Levetiracetam serve as comparison.

### A. The pharmacokinetics of Dextrorotatory oxiracetam in vivo and studies of bioavailability

### 1. Materials

### 1.1 Drugs

### 1.1.1 Standards:

Oxiracetam and dextrorotatory oxiracetam are purchased from Chongqing Dongze Pharmaceutical Science and Technology Co., Ltd., lot number: oxiracetam: 20080917; dextrorotatory oxiracetam: 20100205.

### 1.1.2 Internal Standard:

Internal Standard: Piracetam, lot number 100386-200702 (content 100.0%), purchased from NIFDC.

### 3.2 Reagent

Methanol (MERCK, for liquid chromatography), water (MILLI Q), acetonitrile (TEDIA, for liquid chromatography).

### 3.3 Instrument

SHIMADZU LC-MS single quadrupole mass spectrometer, Eppendorf 5430 desktop high speed centrifuge, XW-80A vortex mixer, SE812 nitrogen blowing instrument and SE812J numerical control constant temperature water bath (Beijing Ferren Science & Technology Co. Ltd.).

### 4 Determination methods of oxiracetam and dextrorotatory oxiracetam in plasma by LC-MS

### 4.1 Chromatographic conditions

Mobile phase: methanol: water (5:95)
Column: ZORBAX SB-Aq (2.1 x 100 mm 3.5 µ)
Flow rate: 0.1 mL/min;
Column temperature: 30°C;
Injection volume: 10 µL

### 4.2 Mass spectrometry detection parameters

Mass spectrometry ionization method: electrospray ionization (ESI); ion polarity Positive; scanning mode for multiple reactions monitoring (MRM); scanning time of 100ms. The ionic reactions used for quantitative analysis were m/z (oxiracetam) m/z 159.0→m/z 113.9 and m/z 143.1→m/z 126.0 (piracetam), respectively.

### 4.3 Preparation of standard solutions

Preparation of oxiracetam standard: oxiracetam standard of 100.01mg is accurately weighed by weight loss method, dissolved with ultrapure water, diluted in 100.0mL volumetric flask, and formulated into 1.0001mg/mL stock solution, and diluted to the appropriate concentration with ultra-pure water upon use.

Preparation of dextrorotatory oxiracetam standard: dextrorotatory oxiracetam standard of 100.01mg is accurately weighed by weight loss method, dissolved with ultrapure water, diluted in 100.0mL volumetric flask, and formulated into 1.0002mg/mL stock solution, and diluted to the appropriate concentration with ultra-pure water upon use.

Internal standard solution: piracetam of 20.02 mg is accurately weighed by weight loss method, dissolved with ultrapure water, diluted in 100.0mL volumetric flask, and formulated into 200.2 mg/mL stock solution, and diluted to the appropriate concentration with ultra-pure water upon use

### 4.4 Extraction of plasma samples

20 µL of the drug-containing the plasma sample is taken in a 1.5 mL Eppendorf tube. 20 µL of piracetam (20 µg/mL) of the internal standard solution is added, and mixed by the vortex mixer for 30s. Then, 500 µL of acetonitrile is added thereto, vortex shaken for 5 min and centrifugated at 16000 rpm for 10 min. The supernatant from the centrifugation was passed through 1 ml of SPE C18E column and the filtrate was collected, put into 38°C constant temperature water bath and dried by nitrogen gas. 300 µL of mobile phase was dissolved and the injection volume was 10 µL.

### 5 Results of Toxicokinetic samples

### 5.1 oxiracetam

### 5.1.1 Preparation of standard solutions and its results

20 µL of blank dog plasma was added into the working solution prepared from the oxiracetam standard. The concentrations of oxiracetam in the plasma were 0.167, 0.33, 0.37, 1.33, 2.67, 4.0, 6.67, 13.33 and 26.67 µg/mL, respectively. A sample was made for each concentration respectively according to the procedures"4.4 Extraction of plasma samples". The ratio of the peak area (As) of oxiracetam to the peak area (Ai) of the internal standard piracetam (f=As/Ai) were calculated, and perform linear regression using the concentration (C) to the ratio (f). The weight parameter 1/x, and the oxiracetam regression equation: y=5.2117X-0.76985 (r=0.9970, weight 1/x).

**Table 1 Data sheet of standard curve of oxiracetam**

| C(µg/ml) | As | Ai | f |
|---|---|---|---|
| 0.167 | 992136 | 16622206 | 0.059687 |
| 0.333 | 1549767 | 16609126 | 0.093308 |
| 0.667 | 3017344 | 10076722 | 0.299437 |
| 1.333 | 5274759 | 11128797 | 0.473974 |
| 2.667 | 9062878 | 12423386 | 0.729501 |
| 4 | 12074304 | 10813359 | 1.11661 |
| 6.667 | 17499548 | 11720309 | 1.493096 |
| 13.33 | 28478007 | 11472202 | 2.482349 |
| 26.67 | 59380130 | 11215251 | 5.294588 |

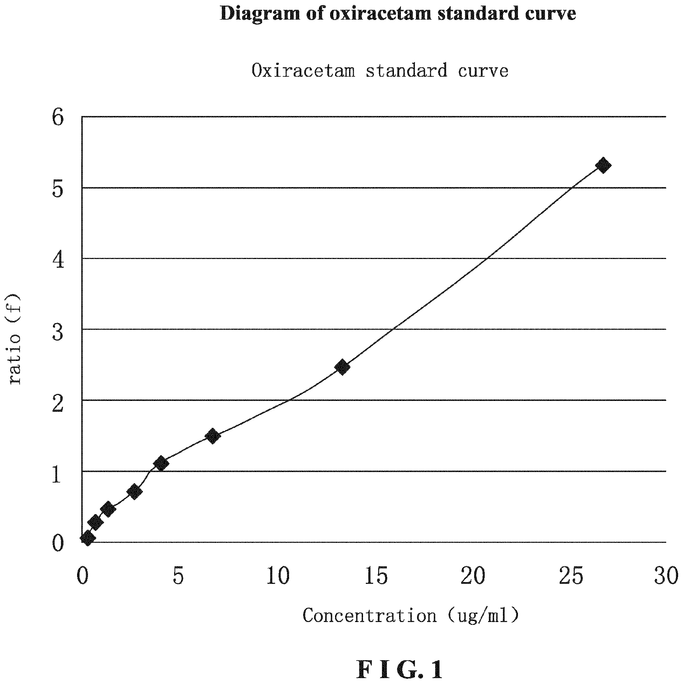

**The diagram of standard curve of oxiracetam is shown as** **Fig. 1****.**

### 5.1.2 The results from the assays of oxiracetam sample

**Table 2 Data sheet of the blood assay of Dog 1-2**

| Time(h) | As | Ai | f | C(µg/ml) | Concentration in dog blood 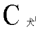 (µg/ml) |
|---|---|---|---|---|---|
| 0 | 0 | 1273566 | 0 | 0 | 0 |
| 0.25 | 276464 | 1315155 | 0.210214 | 0.325722 | 4.885835 |
| 0.5 | 732721 | 1441293 | 0.508378 | 1.879661 | 28.19492 |
| 0.75 | 963865 | 1016862 | 0.947882 | 4.170226 | 62.55338 |
| 1 | 1995011 | 1321985 | 1.509103 | 7.09514 | 106.4271 |
| 1.25 | 1762997 | 1190761 | 1.480563 | 6.946402 | 104.196 |
| 1.5 | 2614193 | 1277486 | 2.046357 | 9.895151 | 148.4273 |
| 2 | 2488246 | 1126617 | 2.2086 | 10.74071 | 161.1106 |
| 4 | 946639 | 1157519 | 0.817817 | 3.492368 | 52.38552 |
| 6 | 370351 | 1451722 | 0.255112 | 0.559715 | 8.39572 |
| 8 | 257446 | 1372566 | 0.187565 | 0.207685 | 3.115275 |
| 12 | 67028 | 768664 | 0.087201 | -0.31539 | -4.7308 |
| 24 | 0 | 987967 | 0 | 0 | 0 |

**Table 3 Data sheet of the blood assay of Dog 1-1**

| Time(h) | As | Ai | f | C(µg/ml) | Concentration in dog blood 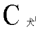 (µg/ml) |
|---|---|---|---|---|---|
| 0 | 0 | 886512 | 0 | 0 | 0 |
| 0.25 | 268473 | 1258959 | 0.21325 | 0.341545 | 5.123175 |
| 0.5 | 902927 | 1326947 | 0.680454 | 2.776474 | 41.64712 |
| 0.75 | 1070002 | 1390229 | 0.769659 | 3.241381 | 48.62071 |
| 1 | 1732865 | 1199276 | 1.444926 | 6.760671 | 101.4101 |
| 1.25 | 1465094 | 1282962 | 1.141962 | 5.181714 | 77.72571 |
| 1.5 | 1378168 | 804776 | 1.712486 | 8.155116 | 122.3267 |
| 2 | 2314341 | 1147313 | 2.017184 | 9.743106 | 146.1466 |
| 4 | 883382 | 1325842 | 0.66628 | 2.702601 | 40.53902 |
| 6 | 252446 | 1240015 | 0.203583 | 0.291164 | 4.367454 |
| 8 | 135200 | 1380872 | 0.097909 | -0.25958 | -3.89365 |
| 12 | 111511 | 1209146 | 0.092223 | -0.28921 | -4.33818 |
| 24 | 0 | 644736 | 0 | 0 | 0 |

**Table 4 Data sheet of the blood assay of Dog 2-6**

| Time(h) | As | Ai | f | C(µg/ml) | Concentration in dog blood 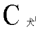 (µg/ml) |
|---|---|---|---|---|---|
| 0 | 0 | 1168679 | 0 | 0 | 0 |
| 0.25 | 88019 | 1042543 | 0.084427 | -0.32984 | -4.94761 |
| 0.5 | 711568 | 1159296 | 0.613793 | 2.429056 | 36.43584 |
| 0.75 | 509831 | 343805 | 1.482907 | 6.958619 | 104.3793 |
| 1 | 1765579 | 1518281 | 1.16288 | 5.290733 | 79.361 |
| 1.25 | 2099611 | 1382506 | 1.518699 | 7.145156 | 107.1773 |
| 1.5 | 1807432 | 1213844 | 1.489015 | 6.99045 | 104.8567 |
| 2 | 896293 | 624506 | 1.435203 | 6.709998 | 100.65 |
| 4 | 677360 | 1308017 | 0.517853 | 1.929042 | 28.93564 |
| 6 | 106646 | 505852 | 0.210825 | 0.328904 | 4.933561 |
| 8 | 87006 | 940861 | 0.092475 | -0.2879 | -4.31848 |
| 12 | 44146 | 1048738 | 0.042094 | -0.55047 | -8.257 |
| 24 | 0 | 644736 | 0 | 0 | 0 |

**Table 5 Data sheet of the blood assay of Dog 2-5**

| Time(h) | As | Ai | f | C(µg/ml) | Concentration in dog blood 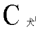 (µg/ml) |
|---|---|---|---|---|---|
| 0 | 0 | 1341040 | 0 | 0 | 0 |
| 0.25 | 266991 | 1093498 | 0.244162 | 0.502651 | 7.539761 |
| 0.5 | 664555 | 969705 | 0.685317 | 2.801815 | 42.02722 |
| 0.75 | 839446 | 1203805 | 0.697327 | 2.86441 | 42.96615 |
| 1 | 576413 | 639882 | 0.900811 | 3.924909 | 58.87363 |
| 1.25 | 1346311 | 1315636 | 1.023316 | 4.563365 | 68.45047 |
| 1.5 | 1685657 | 1215516 | 1.386783 | 6.457647 | 96.86471 |
| 2 | 1344007 | 479583 | 2.802449 | 13.83567 | 207.5351 |
| 4 | 543839 | 783969 | 0.6937 | 2.845504 | 42.68256 |
| 6 | 315813 | 1279837 | 0.24676 | 0.516191 | 7.742862 |
| 8 | 112798 | 1303094 | 0.086562 | -0.31872 | -4.78075 |
| 12 | 130427 | 1187193 | 0.109862 | -0.19728 | -2.95926 |
| 24 | 0 | 1156095 | 0 | 0 | 0 |

**Table 6 Data sheet of the blood assay of Dog 3-3**

| Time(h) | As | Ai | f | C(µg/ml) | Concentration in dog blood 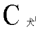 (µg/ml) |
|---|---|---|---|---|---|
| 0 | 0 | 1423765 | 0 | 0 | 0 |
| 0.25 | 65535 | 691180 | 0.094816 | -0.2757 | -4.13545 |
| 0.5 | 874164 | 1282444 | 0.681639 | 2.782649 | 41.73973 |
| 0.75 | 1510853 | 1217024 | 1.241432 | 5.700123 | 85.50185 |
| 1 | 1834880 | 946744 | 1.938095 | 9.330921 | 139.9638 |
| 1.25 | 5184125 | 1291411 | 4.014311 | 20.15153 | 302.273 |
| 1.5 | 2450710 | 939550 | 2.608387 | 12.82428 | 192.3642 |
| 2 | 1717067 | 901121 | 1.905479 | 9.160934 | 137.414 |
| 4 | 544114 | 1304058 | 0.417247 | 1.404715 | 21.07073 |
| 6 | 746143 | 1412032 | 0.528418 | 1.984106 | 29.76158 |
| 8 | 186413 | 1349073 | 0.138179 | -0.0497 | -0.74557 |
| 12 | 91699 | 1373710 | 0.066753 | -0.42195 | -6.32932 |
| 24 | 0 | 1432647 | 0 | 0 | 0 |

**Table 7 Data sheet of the blood assay of Dog 3-4**

| Time(h) | As | Ai | f | C(µg/ml) | Concentration in dog blood 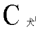 (µg/ml) |
|---|---|---|---|---|---|
| 0 | 0 | 271384 | 0 | 0 | 0 |
| 0.25 | 73940 | 347373 | 0.212855 | 0.339485 | 5.092278 |
| 0.5 | 316630 | 459251 | 0.689449 | 2.82335 | 42.35025 |
| 0.75 | 717070 | 637701 | 1.124461 | 5.090504 | 76.35756 |
| 1 | 3905447 | 1012257 | 3.858158 | 19.33771 | 290.0656 |
| 1.25 | 1355434 | 688449 | 1.968823 | 9.491063 | 142.3659 |
| 1.5 | 877979 | 346678 | 2.532549 | 12.42904 | 186.4355 |
| 2 | 1747729 | 570226 | 3.064976 | 15.20389 | 228.0583 |
| 4 | 145610 | 255476 | 0.569956 | 2.200588 | 33.00882 |
| 6 | 121522 | 478627 | 0.253897 | 0.553385 | 8.300782 |
| 8 | 84930 | 599118 | 0.141758 | -0.03105 | -0.46572 |
| 12 | 104072 | 768305 | 0.135457 | -0.06389 | -0.95836 |
| 24 | 269524 | 634460 | 0.424808 | 1.444124 | 21.66187 |

### 5.2 Dextrorotatory oxiracetam

### 5.2.1 Preparation of standard curves and its results

20 µL of blank dog plasma was added into the working solution prepared from the dextrorotatory oxiracetam standard. The concentrations of dextrorotatory oxiracetam in the plasma were 0.167, 0.33, 0.37, 1.33, 2.67, 4.0, 6.67, 13.33 and 26.67 µg/mL, respectively. A sample was made for each concentration respectively according to the procedures "4.4 Extraction of plasma samples". The ratio of the peak area (As) of dextrorotatory oxiracetam to the peak area (Ai) of the internal standard piracetam (f=As/Ai) were calculated, and perform linear regression using the concentration (C) to the ratio (f). The weight parameter 1/x, and the oxiracetam regression equation: y= 1.6811X-0.031244(r=0.9949, weight 1/x).

**Table 8 Data sheet of standard curve of Dextrorotatory oxiracetam**

| C(µg/ml) | As | Ai | f |
|---|---|---|---|
| 0.067 | 812704 | 16331255 | 0.049764 |
| 0.167 | 1824692 | 17170981 | 0.106266 |
| 0.33 | 3048924 | 17352720 | 0.175703 |
| 0.67 | 3001823 | 11162172 | 0.268928 |
| 1.33 | 5321083 | 7730720 | 0.688304 |
| 2.67 | 8933205 | 6252168 | 1.428817 |
| 4.0 | 12411781 | 4181600 | 2.968189 |
| 6.67 | 17957473 | 4249716 | 4.22557 |
| 13.33 | 29669086 | 3881626 | 7.643468 |

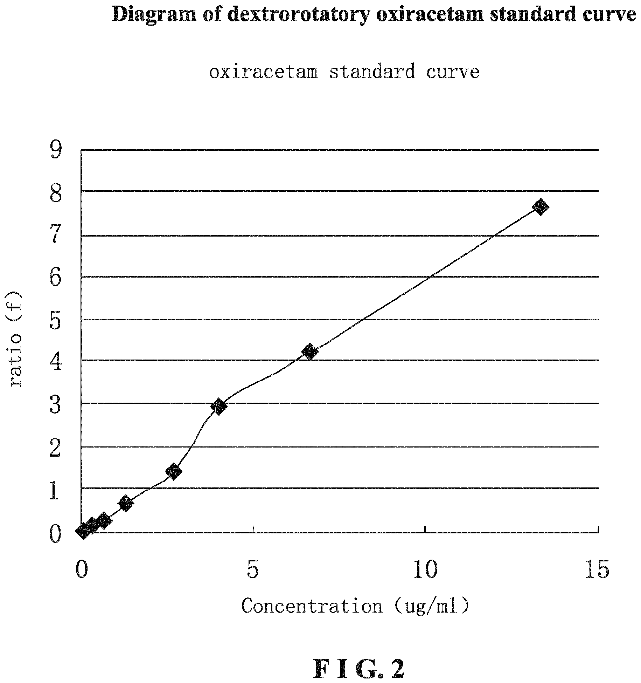

The diagram of standard curve of dextrorotatory oxiracetam is shown as Fig. 2.

### 5.2.2 The results from the assays of oxiracetam sample

**Table 9 Data sheet of the blood assay of Dog 1-5**

| Time(h) | As | Ai | f | C(µg/ml) | Concentration in dog blood 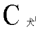 (µg/ml) |
|---|---|---|---|---|---|
| 0 | 0 | 551666 | 0 | 0 | 0 |
| 0.25 | 480732 | 1255106 | 0.383021 | 0.612653 | 9.18979 |
| 0.5 | 1166819 | 952801 | 1.22462 | 2.027464 | 30.41197 |
| 0.75 | 2460020 | 1277065 | 1.926308 | 3.207072 | 48.10608 |
| 1 | 2590580 | 1272425 | 2.035939 | 3.391373 | 50.8706 |
| 1.25 | 1853588 | 485827 | 3.815325 | 6.382699 | 95.74049 |
| 1.5 | 3877552 | 883340 | 4.389648 | 7.348194 | 110.2229 |
| 2 | 1009044 | 297782 | 3.388533 | 5.665218 | 84.97827 |
| 4 | 866497 | 1160418 | 0.746711 | 1.224052 | 18.36078 |
| 6 | 274483 | 853889 | 0.32145 | 0.509146 | 7.637195 |
| 8 | 107211 | 651065 | 0.16467 | 0.245583 | 3.683746 |
| 12 | 72049 | 873862 | 0.082449 | 0.107361 | 1.610414 |
| 24 | 0 | 1161598 | 0 | 0 | 0 |

**Table 10 Data sheet of the blood assay of Dog 1-6**

| Time(h) | As | Ai | f | C(µg/ml) | Concentration in dog blood 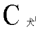 (µg/ml) |
|---|---|---|---|---|---|
| 0 | 0 | 672215 | 0 | 0 | 0 |
| 0.25 | 60261 | 488100 | 0.12346 | 0.176305 | 2.644578 |
| 0.5 | 359624 | 956443 | 0.376001 | 0.600852 | 9.012782 |
| 0.75 | 844158 | 1176092 | 0.717765 | 1.175391 | 17.63087 |
| 1 | 1618357 | 1132934 | 1.428465 | 2.370149 | 35.55224 |
| 1.25 | 1855341 | 1265601 | 1.465976 | 2.433209 | 36.49813 |
| 1.5 | 2010997 | 1322332 | 1.520796 | 2.525366 | 37.88049 |
| 2 | 3237253 | 1200625 | 2.696307 | 4.501517 | 67.52275 |
| 4 | 835318 | 901936 | 0.926139 | 1.525688 | 22.88532 |
| 6 | 170377 | 295516 | 0.576541 | 0.937979 | 14.06968 |
| 8 | 131752 | 638978 | 0.206192 | 0.315385 | 4.730775 |
| 12 | 86278 | 913399 | 0.094458 | 0.12755 | 1.913244 |
| 24 | 0 | 1045161 | 0 | 0 | 0 |

**Table 11 Data sheet of the blood assay of Dog 2-3**

| Time(h) | As | Ai | f | C(µg/ml) | Concentration in dog blood 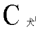 (µg/ml) |
|---|---|---|---|---|---|
| 0 | 0 | 751840 | 0 | 0 | 0 |
| 0.25 | 702738 | 1144185 | 0.614182 | 1.001258 | 15.01886 |
| 0.5 | 2005286 | 981191 | 2.043726 | 3.404465 | 51.06697 |
| 0.75 | 2432070 | 813496 | 2.989652 | 4.99466 | 74.9199 |
| 1 | 3448056 | 1222672 | 2.820099 | 4.709624 | 70.64436 |
| 1.25 | 3208841 | 1124874 | 2.852623 | 4.7643 | 71.4645 |
| 1.5 | 3021390 | 992719 | 3.04355 | 5.085268 | 76.27902 |
| 2 | 3072138 | 1172934 | 2.619191 | 4.371878 | 65.57817 |
| 4 | 750345 | 1001486 | 0.749232 | 1.228289 | 18.42434 |
| 6 | 236168 | 469767 | 0.502734 | 0.813903 | 12.20854 |
| 8 | 116823 | 309924 | 0.376941 | 0.602431 | 9.036468 |
| 12 | 92460 | 871874 | 0.106047 | 0.147032 | 2.205485 |
| 24 | 0 | 465528 | 0 | 0 | 0 |

**Table 12 Data sheet of the blood assay of Dog 2-4**

| Time(h) | As | Ai | f | C(µg/ml) | Concentration in dog blood 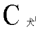 (µg/ml) |
|---|---|---|---|---|---|
| 0 | 0 | 1221788 | 0 | 0 | 0 |
| 0.25 | 114587 | 1174751 | 0.097542 | 0.132733 | 1.990996 |
| 0.5 | 309169 | 961759 | 0.321462 | 0.509166 | 7.637487 |
| 0.75 | 769661 | 750649 | 1.025327 | 1.692434 | 25.38651 |
| 1 | 646555 | 335063 | 1.929652 | 3.212694 | 48.19041 |
| 1.25 | 1517117 | 774517 | 1.958791 | 3.26168 | 48.9252 |
| 1.5 | 1556348 | 523101 | 2.975234 | 4.970422 | 74.55633 |
| 2 | 1796540 | 589667 | 3.046703 | 5.090568 | 76.35852 |
| 4 | 605421 | 743917 | 0.813829 | 1.336883 | 20.05325 |
| 6 | 219353 | 1081137 | 0.202891 | 0.309836 | 4.647542 |
| 8 | 130321 | 1179320 | 0.110505 | 0.154526 | 2.317895 |
| 12 | 68710 | 897659 | 0.076544 | 0.097433 | 1.4615 |
| 24 | 0 | 1202856 | 0 | 0 | 0 |

**Table 13 Data sheet of the blood assay of Dog 3-1**

| Time(h) | As | Ai | f | C(µg/ml) | Concentration in dog blood 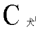 (µg/ml) |
|---|---|---|---|---|---|
| 0 | 0 | 58780 | 0 | 0 | 0 |
| 0.25 | 278520 | 782480 | 0.355945 | 0.567135 | 8.507032 |
| 0.5 | 257902 | 194291 | 1.327401 | 2.200249 | 33.00374 |
| 0.75 | 668626 | 333408 | 2.005429 | 3.340082 | 50.10123 |
| 1 | 2113431 | 965118 | 2.189816 | 3.650056 | 54.75084 |
| 1.25 | 2462010 | 1055741 | 2.332021 | 3.889116 | 58.33674 |
| 1.5 | 2457765 | 993482 | 2.47389 | 4.127612 | 61.91418 |
| 2 | 1235252 | 428685 | 2.881491 | 4.812831 | 72.19246 |
| 4 | 1231620 | 1949894 | 0.631634 | 1.030596 | 15.45895 |
| 6 | 259097 | 616016 | 0.420601 | 0.675828 | 10.13743 |
| 8 | 233363 | 1082583 | 0.215561 | 0.331136 | 4.967042 |
| 12 | 103250 | 644955 | 0.160089 | 0.237881 | 3.568216 |
| 24 | 0 | 1202856 | 0 | 0 | 0 |

**Table 14 Data sheet of the blood assay of Dog 3-2**

| Time(h) | As | Ai | f | C(µg/ml) | Concentration in dog blood 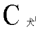 (µg/ml) |
|---|---|---|---|---|---|
| 0 | 0 | 508668 | 0 | 0 | 0 |
| 0.25 | 1172419 | 478962 | 2.447833 | 4.083808 | 61.25712 |
| 0.5 | 746835 | 250642 | 2.979688 | 4.97791 | 74.66865 |
| 0.75 | 2335279 | 1024693 | 2.279004 | 3.799989 | 56.99983 |
| 1 | 1264281 | 283050 | 4.466635 | 7.477616 | 112.1642 |
| 1.25 | 975731 | 349361 | 2.792902 | 4.663903 | 69.95855 |
| 1.5 | 1414091 | 604412 | 2.339614 | 3.901882 | 58.52823 |
| 2 | 874924 | 572365 | 1.528612 | 2.538506 | 38.07758 |
| 4 | 616581 | 1189738 | 0.518249 | 0.839985 | 12.59978 |
| 6 | 226864 | 1172950 | 0.193413 | 0.293903 | 4.408544 |
| 8 | 129261 | 1214800 | 0.106405 | 0.147634 | 2.214506 |
| 12 | 81538 | 1141148 | 0.071453 | 0.088875 | 1.333125 |
| 24 | 0 | 588337 | 0 | 0 | 0 |

The major pharmacokinetic parameters of oxiracetam and dextrorotatory oxiracetam in dogs are as follow: Tmax were (0.022 ± 0.131), (2.039 ± 0.401), (16.37 ± 0.408) h, respectively, and the Cmax were (202.383 ±79.525), (85.790±19.953) mg/ml, T1/h; AUC0-∞ were (445.340 ± 86.609), (242.262 ± 31.343) mg*h/ml, respectively. The relative bioavailability of dextrorotatory oxiracetam was (112.2 ± 11.6) % with oxiracetam as a reference. There was no significant difference between the nonparametric test results (P> 0.05).

### B. Toxicological test

The mouse toxicity of dextrorotatory oxiracetam and oxiracetam was compared under GLP assay conditions. The dose is set to 5g/kg. Each group of 10 animals, with 18.3 ± 1.5 grams of weight, half male and half female, were orally administrated with the prepared 0.5% CMC liquid. The results showed that the two groups had normal activity and no obvious toxicity. There was no significant difference in toxicity between the two groups.

Oxiracetam is commercially available. Its properties on pharmakinetics and toxicology are well-known.

### C. Experimental study of Dextrorotatory oxiracetam against epilepsy

The rat epilepsy models were treated with lithium chloride-pilocarpine and used levetiracetam as positive control. The duration of acute seizures of lithium chloride-pilocarpine epilepsy model rat was selected as the observation time, and prophylactic administration was used.

### 1 Experimental Material and Systems

### 1.1 Experimental Materials

Testing sample:
Dextrorotatory oxiracetam, raw material, milky white powder, lot number 20100205, soluble in water.
levorotatory oxiracetam, white powder, lot number 091202, soluble in water.

The above were purchased from Chongqing Dongze Pharmaceutical Science and Technology Co., Ltd.

Positive control:
Levetiracetam tablet is used in plus treatment for adults and children over 4 years of age epilepsy patients with partial seizure, having traits for blue oval film coated tablets, 0.25g/tablet, and manufactured by UCB Pharma S.A. The initial dose of an adult is 500mg/times, twice daily, that is, 1000mg/60kg, 16.7mg/kg. The dose of a rat is converted to 100mg / kg according to body surface area.
S-oxiracetam and dextrorotatory oxiracetam were prepared with pure water before use. The levetiracetam tablet was prepared with 0.5% CMC at the desired concentration before use. 0.5% CMC is used as solvent reference substance, lot number 2014010801, manufactured by Chengdu Kelong Chemical Reagent Factory.

Reagents:
Nitrochrome pilocarpine (pilocarpine) eye drops, 5ml: 25mg, lot number: 14101901, Shandong Dr. Lun Fu Ruida Pharmaceutical Co., Ltd, diluted to 3mg/ml of concentration with NS before use.
Anhydrous lithium chloride, white particles, molecular weight 42.39, Chengdu Kelong Chemical Reagent Factory, prepared with NS to 12.7 mg/ml as spare.

### 1.2 Laboratory system

Laboratory animals
Species/strains: SD rats
Level: SPF level
Supply unit: Chengdu Dasuo Biological Technology Co., Ltd
Production license number: SCXK (Sichuan) 2008-24
Purchase of animal sex and quantity: 50, half male and half female;
Weight range when purchased: 120 ∼ 150g
Animal feeding and management
Environmental conditions for animal feeding and management
   Feeding room: barrier system animal laboratory
   Environmental class: barrier system
Temperature: 20 ∼ 25 °C
Relative humidity: 40 ∼ 70%;
Number of ventilation: 10-15times/hour
Lighting time: 12h / 12h alternating lighting every day.
Animal cages: PC polycarbonate cartridges (L×W×H: 460 mm×315 mm×210 mm), by the Suzhou City, Suzhou and Hangzhou animal plant production.
Litter: the corncob as litter, provided by the Suzhou Shuang Lion Experimental Animal Feed Technology Co., Ltd., heated by high temperature and high pressure steam sterilization for animal use.
Cage updates frequency: 2-3 times a week to replace.
Cleaning and disinfection: cleaning was carried out after the end of daily test operation and feeding work, and then disinfection by the animal management room. Disinfectants were alternately selected 84 disinfectants, Lysol and bromogeramine, once a week to exchange the type of disinfectants.

### Quarantine

The quarantine period is set to 7 days. During the quarantine period, the daily appearance and the general state of the animals are observed and recorded. If any animals acted abnormal, the test may be affected. The animal should be removed from the test and shall not be used.

### Fodder

Rat full price pellet feed provided by the Institute of Laboratory Animals of Sichuan Academy of Medical Science & Sichuan Provincial Research' s Hospital, which were meet GB14924.3-2001 standard were used after radiation of Co60.
Feeding method: free intake
Drinking water

High pressure steam sterilization tap water was freely taken by animal drinking water bottles.

### 2 Experiment methods

### 2.1 Animal identification and numbering

The tail marking method was used during the quarantine period, that is, the animal mark is indicated by the mark stroke on the tail. Prior to administration, the animals were grouped by body weight stratification randomized grouping and labeled with picric acid staining.

### 2.2 Dose setting

Animals were randomly divided into 4 groups, 10/group. Levetiracetam was selected as a positive control, 1.5 times of clinical dose were chosen, that is, 150 mg/kg. The clinical dose of an adult is 800 mg/times, twice daily, that is, 1600 mg/60kg, 26.7 mg/kg. The dose of a rat is converted to 160 mg/kg according to body surface area. Dextrorotatory oxiracetam and levorotatory oxiracetam were selected half of the dose, that is, 80 mg/kg, and 1.5 times is 120 mg/kg. The dose was 1 ml/100 g, and the rats were treated with continuous administration for 7 days. As levetiracetam administration of 1.3h (80min), the plasma concentration peak, so we choose the last 80 minutes after the last administration of modeling. As administration of levetiracetam for 1.3h (80min), the plasma concentration peak was reached, so each group was selected after the last 80 minutes after the start of modeling.

The specific dose settings are as follows:

| Groups | Serial number of each dog | dose (mg/kg) | Concentration (mg/ml) | Clinical dose multiple |
|---|---|---|---|---|
| Model Control Group | 1F01∼1F05. 1M01∼1M05 | - | 0.5%CMC | - |
| Levetiracetam Group | 2F01∼2F05, 2M01∼2M05 | 150 | 15 | 1.5 |
| S-Oxiracetam Group | 3F01∼3F05, 3M01∼3M05 | 120 | 12 | 1.5 |
| Dextrorotatory Oxiracetam Group | 4F01∼4F05, 4M01∼4M05 | 120 | 12 | 1.5 |

### 2.3 Test methods

Modeling method: After several previous test, the following modeling method was chosen: Animal ip. Lithium chloride 3 mmol/kg (1.5 ml/L) 127 mg/kg (12.7 mg/ml, 1 ml/100g body weight), 24h, 37.5 mg/kg pilocarpine three times ip. mg/kg (2.5 mg/ml, 0.5 ml/100 g body weight) at intervals of 10 minutes.

As the number of animals per observation was limited, the test was divided into four batches.

### 2.4 Observe indicators

The seizure status, seizure level, latency, duration of seizures, seizures and mortality were recorded within 3 hours after modeling, and compared with the model control group,

### 3 Test results

All rats had symptoms of cholinergic peripheral nerve stimulation, that is, vertical hair, salivation, blood tears, after 5 to 10 min of the first injection of pilocarpine. Also, varying degrees of the following performance appeared, including: less moving, stare, mouth automatic disease, nod, and blink, wet dog-like shaking.

### 3.1 Mortality rate and number of SE Animals

| Groups | Numbers of Animals | Dose (mg/kg) | Number of Deaths | Mortality rate (%) | Number of SE Animals | SE occurrence rate (%) |
|---|---|---|---|---|---|---|
| Model Control Group | 10 | - | 4 | 40 | 6 | 60 |
| Levetiracetam group | 10 | 150 | 5 | 50 | 7 | 70 |
| S-Oxiracetam Group | 10 | 120 | 3 | 30 | 4 | 40 |
| Dextrorotatory Oxiracetam Group | 10 | 120 | 1 | 10 | 2 | 20 |

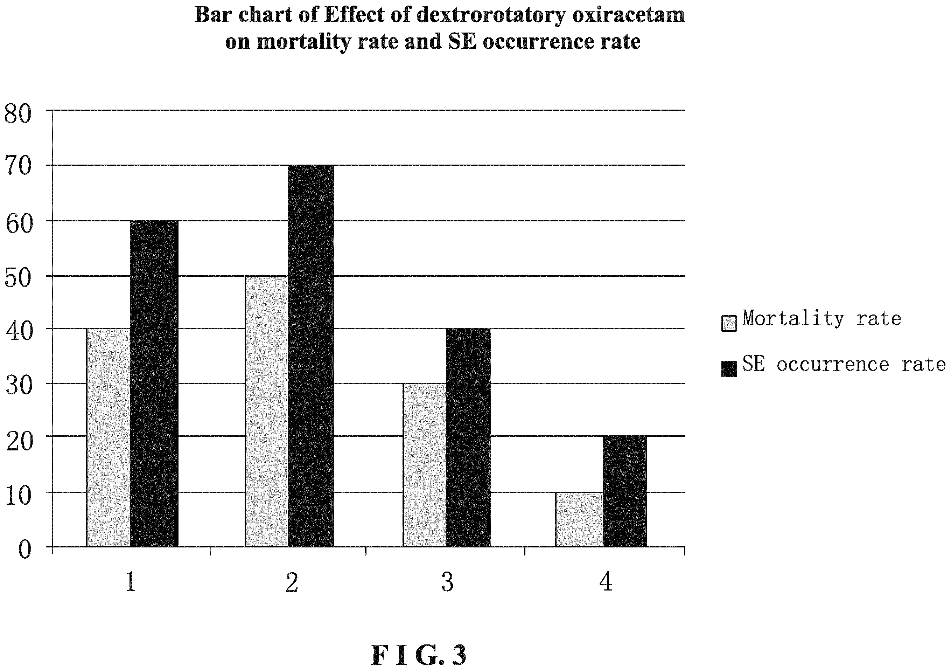

Effect of dextrorotatory oxiracetam on mortality rate and SE occurrence rate to the lithium chloride-pilocarpine-made epilepsy model rats is shown as Fig.3.

The results of the above test showed that the animals in each group appeared varying degrees of alkali-related peripheral nerve stimulation symptoms and different levels of convulsions. There was a certain mortality rate within 1 to 7 days after modeling. The mortality was 40% for the model control group, 50% for the levetiracetam group, 30% for the S-oxiracetam group and 10% for the dextrorotatory oxiracetam group. The percentages of status epilepticus in each group were 60%, 70%, 40%, 20%. The percentage of SE in dextrorotatory oxiracetam animals was significantly lower than in the control group. It is indicated that dextrorotatory oxiracetam could reduce the percentage of epilepsy in pilocarpine epilepsy model rats and the mortality rate, and had protective effect on epilepsy rats.

### 3.2 Different stages of animals

| Groups | Number of animals | Dose (mg/kg) | Occurrence rate (%) | | | | |
|---|---|---|---|---|---|---|---|
| | | | Stage I | Stage II | Stage III | Stage IV | Stage V |
| Model Control Group | 10 | - | 10 | 10 | 9 | 8 | 6 |
| Levetiracetam group | 10 | 150 | 10 | 9 | 8 | 7 | 7 |
| S-Oxiracetam Group | 10 | 120 | 10 | 10 | 5 | 4 | 4 |
| Dextrorotatory Oxiracetam Group | 10 | 120 | 10 | 10 | 5 | 2* | 2 |

| | | | | | | | |
|---|---|---|---|---|---|---|---|
| Note: * indicates that the model control group by X² test, P <0.05 | | | | | | | |

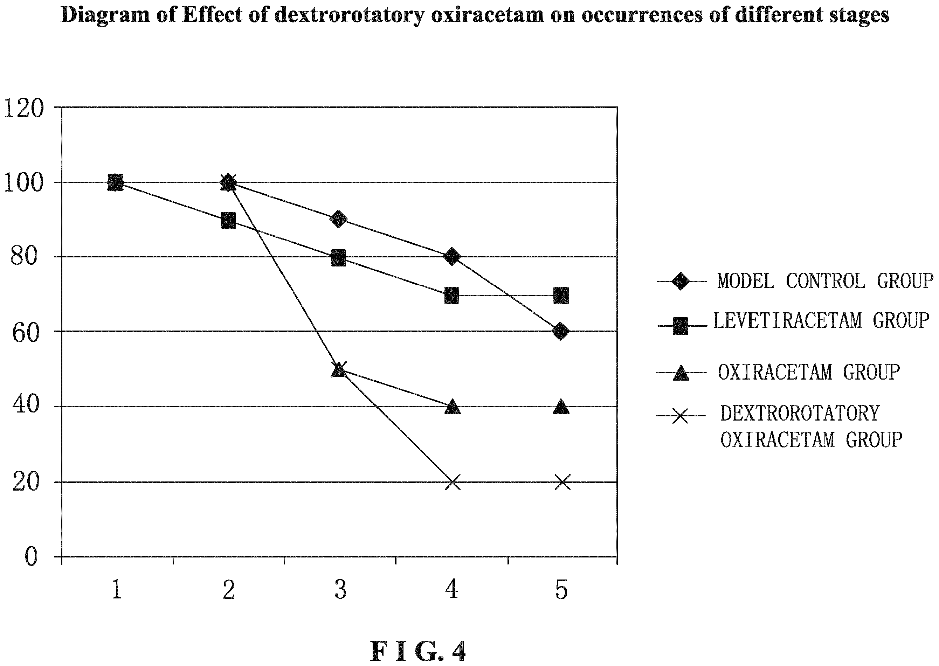

The effect of dextrorotatory oxiracetam on occurrences of different stages is shown in Fig.4.

In the above tests, all the animals in the group had all the symptoms of stage I and II, half of the S-oxiracetam group and dextrorotatory oxiracetam group had no symptoms of stage III and above. The number of animals having stage IV in the dextrorotatory oxiracetam group was 20%, significantly different from the model control group (P <0.05). It is indicated that dextrorotatory oxiracetam group can significantly reduce the number of animals having symptoms of stage IV in the model rats and inhibit seizures.

### 3.3 Latency of different stages

| Groups | Number of animals | Dose (mg/kg) | Latency (min) | | | | |
|---|---|---|---|---|---|---|---|
| | | | Stage I | Stage II | Stage III | Stage IV | Stage V |
| Model Control Group | 10 | - | 14.3±5.3 | 22.5±5.3 | 35.9±7.0 | 39.6±9.5 | 73.5±24.2 |
| Levetiracetam group | 10 | 150 | 17.6±4.1 | 22.6±5.8 | 34.6±9.4 | 33.6±8.3 | 74.3±24.3 |
| S-Oxiracetam Group | 10 | 120 | 16.9±5.4 | 26.7±6.5 | 35.4±5.8 | 37.8±7 .4 | 64.5±23.2 |
| Dextrorotatory Oxiracetam Group | 10 | 120 | 19.6±4.4 | 34.7±15.2 | 40.2±12.1 | 35.0±2.8 | 38.0±2.8 |

Experimental data showed that the animals in the dextrorotatory oxiracetam group appear the trend for longer latency of stage I, II and III symptoms than the model control group. It is indicated that dextrorotatory oxiracetam can prolong the time of partial epileptic seizures of the model animals, and has protective effects on epilepsy model rats.

### 3.4 Number of seizures and duration at different stages

| Group | Numbers of Animals | Dose (mg/kg) | Number of seizures (times) | | | duration (min) | |
|---|---|---|---|---|---|---|---|
| | | | Stage I | Stage II | Stage III | Stage IV | Stage V |
| Model Control Group | 10 | - | 2.0+1.6 | 6.9±5.3 | 4.7±2 2 | 16.5±15.2 | 72.0±14.4:(*) |
| Levetiracetam group | 10 | 150 | 1.8±0.8 | 4.4±3.7 | 3.6±2.3 | 23.3±6.6 | 63.3±19.0 |
| S-Oxiracetam Group | 10 | 120 | 2.2±1.0 | 5.2±3.1 | 5.6±4.2 | 27.5±12.1 | 79.8±29.5 |
| Dextrorotatory Oxiracetam Group | 10 | 120 | 2.3±1.2 | 6.8±3.7 | 6.4±3.6 | 10.0±1.4 | 26.5±0.7* |

| | | | | | | | |
|---|---|---|---|---|---|---|---|
| Note: * indicates that the model control group by analysis of variance, P <0.05 | | | | | | | |

The duration of stage V seizures of animals in the dextrorotatory oxiracetam group was significantly lower than that in the control group (P <0.05). It is indicated that dextrorotatory oxiracetam group can significantly reduce the timer of stage IV symptoms occurred on the model rats, and had protective effects on epilepsy model rats inhibit seizures.

**Pentetrazol (PTZ) was used to ignite the rat epilepsy model with Sodium valproate as a positive control, in order to observe whether dextrorotatory oxiracetam has the antiepileptic effect.**

### 1. Testing sample, reference substances and reagents

### Testing sample:

Dextrorotatory oxiracetam, raw materials, white powder, lot number 20150603, content:99.92%, soluble in water. provide by Chengdu Biotoppharma Medical Technology Co., Ltd.

### Positive control:

Sodium valproate, lot number 140440, specified as 0.5g / tablet, Sanofi Hangzhou Pharmaceutical Co., Ltd. Gastrointestinal absorption of oral administration was quick and complete. The peak plasma concentration was reached after the administration about 1 to 4 hours, with bioavailability of nearly 100%. The daily maximum dose for human is 30 mg/kg, and for a rat dose is 185mg/kg by conversion of body surface area.

Dextrorotatory oxiracetam was prepared by pure water before use. Sodium valproate tablets ewas formulated by 0.5% CMC into the desired concentration of the suspension solution before use. 0.5% CMC is used as reference substance of vehicles, lot number 2014010801, Chengdu Kelong Chemical Reagent Factory.

### Reagents:

Pentetrazol (PTZ), Sigma, MKBT2860V, white powder, prepared with NS into a concentration of 7mg / ml and reserved before use

### 2. Laboratory system

### 2.1 Laboratory animals

Species/strains: SD rats
Level: SPF level
Supply unit: Chengdu Dasuo Biological Technology Co., Ltd
Production license number: SCXK (Sichuan) 2008-24
Purchase of animal sex and quantity: 40, male;
Weight range when purchased: 120∼150g

### 2.2 Animal feeding and management

### 2.2.1 Environmental conditions for animal feeding and management

Feeding room: barrier system animal laboratory
Environmental class: barrier system
Temperature: 20 ∼ 25 °C
Relative humidity: 40 ∼ 70%;
Number of ventilation: 10-15times/hour
Lighting time: 12h / 12h alternating lighting every day.
Animal cages: PC polycarbonate cartridges (L×W×H: 460 mm×315 mm×210 mm), by the Suzhou City, Suzhou and Hangzhou animal plant production.
Litter: the corncob as litter, provided by the Suzhou Shuang Lion Experimental Animal Feed Technology Co., Ltd., heated by high temperature and high pressure steam sterilization for animal use.
Cage updates frequency: 2-3 times a week to replace.
Cleaning and disinfection: cleaning was carried out after the end of daily test operation and feeding work, and then disinfection by the animal management room. Disinfectants were alternately selected 84 disinfectants, Lysol and bromogeramine, once a week to exchange the type of disinfectants.

### 2.2.2 Quarantine

The quarantine period is set to 7 days. During the quarantine period, the daily appearance and the general state of the animals are observed and recorded. If any animals acted abnormal, the test may be affected. The animal should be removed from the test and shall not be used.

### 2.2.3 Fodder

Rat full price pellet feed provided by the Institute of Laboratory Animals of Sichuan Academy of Medical Science & Sichuan Provincial Research' s Hospital, which were meet GB14924.3-2001 standard were used after radiation of Co60.
Feeding method: free intake

### 2.2.4 Drinking water

High pressure steam sterilization tap water was freely taken by animal drinking water bottles.

### 3 Experiment methods

### 3.1 Animal identification and numbering

The tail marking method was used during the quarantine period, that is, the animal mark is indicated by the mark stroke on the tail. Prior to administration, the animals were grouped by body weight stratification randomized grouping and labeled with picric acid staining.

### 3.2 Modeling method

Rats were injected intraperitoneally with PTZ at a dose of 35 mg/kg (7 mg/ml, 0.5 ml/100 g body weight) for sub-convulsions three times weekly. The animals are weighed before each ip, and observed for behavioral changes after ip. within 1-2h. Behavioral scoring was carried out according to the revised Racine score. Three consecutive occurrences of seizures of stage IV and above indicate successful ignition.

According to the previous tests, generally in the tenth time of the PTZ injection, three seizures of stage IV occur consecutively, means successful modeling.

### 3.3 Dose setting

The animals successfully ignited were chosen to be grouped. The animals were randomly divided into 4 groups, 8-10/group. Sodium valproate was selected as a positive control. 3 times of clinical dose were chosen, that is, 500 mg/kg. According to the results of the previous tests, 120mg / kg was selected as the low dose of dextrorotatory oxiracetam, and 3 times thereof, 360mg / kg as the high dose. The dosage is 1ml/100g, continuous intragastrical administration of 10 days. Specific dose settings are as follows:

**Table 1 Dose Settings**

| Groups | dose (mg/kg) | Concentration (mg/ml) | Clinical dose multiple |
|---|---|---|---|
| Model Control Group | - | 0.5%CMC | - |
| Sodium valproate Control Group | 500 | 50 | 3 |
| Low-dose Dextrorotatory Oxiracetam Group | 120 | 12 | - |
| High-dose Dextrorotatory Oxiracetam Group | 360 | 36 | - |

### 3.4 Test methods

One hour after administration of each group of the animals (according to the peak time of sodium valproate) ip. PTZ 35mg / kg. The animals are observed for behavioral changes within 2h. Behavioral scoring was carried out according to the revised Racine score, continuous administration for 10 days, grading standards as mentioned above.

### 3.5 Observe indicators

The seizures were recorded daily ip PTZ within 2h. The seizure levels, latencies, mortality were recorded, and compared with the model control group.

### 4 Test results

### 4.1 Model selection

A total of 41 animals died during the animal modeling. Twenty-seven animals that were successfully ignited were screened and grouped according to the seizure stages, with about 7 animals in each group.

### 4.2 Mortality rate

During the administration, two animals died in the model group, with a mortality rate of 25%. No animal died in the low-dose group of dextrorotatory oxiracetam and in the high-dose group and the positive control group. The chi-square test, no statistical difference.

**Table 2 Impact on Mortality**

| Groups | Numbers of animals (Number) | Dose ( mg/kg ) | Numbers of deaths (Number) | mortality rate (%) |
|---|---|---|---|---|
| Model Control Group | 8 | - | 2 | 25 |
| Sodium valproate control group | 6 | 500 | 0 | 0 |
| Low-dose Dextrorotatory Oxiracetam Group | 7 | 120 | 1 | 14.3 |
| High-dose Dextrorotatory Oxiracetam Group | 6 | 360 | 0 | 0 |

### 4.3 Occurrence rates of stage IV or above seizures

**Table 3-1 occurrence rates of stage IV or above seizures (%)**

| Groups | Dose (mg/kg) | D1 | D2 | D3 | D4 | D5 |
|---|---|---|---|---|---|---|
| Model control group | - | 7/8 (87.5) | 6/7(85.7) | 5/7 (71.4) | 5/6 (83.3) | 5/6 (83.3) |
| Sodium valproate control group | 500 | 4/6 (66.7) | 3/6(50.0) | 3/6 (50.0) | 2/6 (33.3) | 2/6 (33.3) |
| Low-dose Dextrorotatory Oxiracetam Group | 120 | 4/7 (57.1) | 4/6(66.7) | 3/6 (50.0) | 2/6 (33.3) | 2/6 (33.3) |
| High-dose Dextrorotatory Oxiracetam Group | 360 | 4/6 (66.7) | 3/6 (50.0) | 3/6 (50.0) | 2/6 (33.3) | 1/6 (16.7) |

**Table 3-2 Occurrence rates of animal with Stage IV or above seizures (%)**

| Groups | Dose ( mg/kg) | D6 | D7 | D8 | D9 | D10 |
|---|---|---|---|---|---|---|
| Model control group | - | 5/6 (83.3) | 5/6 (83.3) | 5/6 (83.3) | 4/6 (66.7) | 4/6 (66.7) |
| Sodium valproate control group | 500 | 2/6 (33.3) | 1/6* (16.7) | 0/6** (0) | 1/6 (16.7) | 0/6* (0) |
| Low-dose Dextrorotatory Oxiracetam Group | 120 | 2/6 (33.3) | 1/6* (16.7) | 1/6* (16.7) | 0/6* (0) | 0/6* (0) |
| High-dose Dextrorotatory Oxiracetam Group | 360 | 1/6 (16.7) | 0/6** (0) | 0/6** (0) | 0/6* (0) | 0/6* (0) |

| | | | | | | |
|---|---|---|---|---|---|---|
| Note: Compared with the model control group, by chi-square test, * indicates P <0.05; ** indicates P <0.01. | | | | | | |

The results are shown in Tables 3-1, 3-2 and 5. The results showed that during the administration of the model group animals, the frequency of symptoms of stage IV or above remained at a high level (80%). Compared with the model group, there was no significant difference among the three groups in the early administration. From D7 (D is the number of days, D7 is the seventh day), the occurrence rate was significantly reduced until the end of administration, with statistical differences. The high-dose dextrorotatory Oxiracetam group of animals from D7 have only stage II-III seizures, and no stage IV, V seizures occurs. From the occurrence rate in the diagram can also be seen that the seizures of stage IV and V of the three administration group was significantly lower than the model control group, the high-dose dextrorotatory Oxiracetam group has the best effect.

### 4.4 Latency

The results are shown in Table 4-1 and 4-2. In the early administration, each administration group compared with the model group, the onset of latency was no significant difference. Starting from D4, the latency of animals in the positive group and the high-dose dextrorotatory Oxiracetam group was significantly prolonged (P <0.05, P <0.01), and continued until the end of administration. Low-dose dextrorotatory Oxiracetam group starting from D6, the latency was significantly longer than the model control group (P <0.05, P <0.01), until the end of administration.

**Table 4-1 Seizure latency data (m, X±SD)**

| Groups | Dose ( mg/kg) | D1 | D2 | D3 | D4 | D5 |
|---|---|---|---|---|---|---|
| Model Control Group | - | 3.88±1.25 | 4.25±0.71 | 4.25±1.04 | 4.00±0.76 | 4.38±0.92 |
| Sodium valproate Control Group | 500 | 4.00±1.41 | 5.67±2.25 | 5.67±1.37 | 6.33±1.63* | 6.83±1.47** |
| Low-dose Dextrorotatory Oxiracetam Group | 120 | 3.57±1.51 | 4.57±1.72 | 5.00±1.15 | 4.86±1.07 | 5.43±0.98 |
| High-dose Dextrorotatory Oxiracetam Group | 360 | 3.83±1.17 | 5.00±0.89 | 6.17±1.17 | 6.50±1.05* | 7.17±0.75* |

**Table 4-2 Seizure latency data (m, X±SD)**

| Groups | Dose ( mg/kg) | D6 | D7 | D8 | D9 | D10 |
|---|---|---|---|---|---|---|
| Model Control Group | - | 4.75±1.04 | 4.63±0.92 | 4.88±0.83 | 4.38±0.74 | 4.75±0.71 |
| Sodium valproate Control Group | 500 | 7.33±1.03 ** | 7.50±1.05 ** | 7.83±0.75 ** | 7.67±0.82* * | 8.33±0.82** |
| Low-dose Dextrorotatory Oxiracetam Group | 120 | 6.29±1.11 * | 6.86±1.07 ** | 7.14±1.07 ** | 7.71±0.76* * | 8.43±0.98** |
| High-dose Dextrorotatory Oxiracetam Group | 360 | 7.83±0.75 ** | 8.33±1.03 ** | 8.83±0.98 ** | 8.83±0.75* * | 9.83±0.75** |

| | | | | | | |
|---|---|---|---|---|---|---|
| Note: Compared with the model control group, one-way analysis of variance, * means P <0.05; ** means P <0.01. | | | | | | |

The results showed that after modeling, there were different degrees of alkali peripheral nerve stimulation symptoms and different levels of seizures in each group of animals, and there was a certain mortality (3/41) during the modeling. Animals were also killed during the dosing period, with a mortality rate of 25% in the model control group, 0 in the positive control group, 14.3% in the low-dose dextrorotatory Oxiracetam group, 0 in the high-dose dextrorotatory Oxiracetam group, Sodium soda group mortality rate of 0. In absolute values, it has a protective effect on seizures in animals.

On the stage IV or above seizures and the latency of the results, the dextrorotatory Oxiracetam and positive drugs on the epilepsy model have significant protective effects, the performance in the middle and late administration of the stage IV seizures were significantly lowered, the latency was significantly longer, of which the role of the high dose dextrorotatory Oxiracetam was most obvious.

The test results showed that the dextrorotatory Oxiracetam has a curative effect on the epilepsy model, the performance in the mortality rate and the stage IV or above seizures were lower than the model control group, and the latency was significantly longer than the model control.

The invention has the advantages that:
The inventor accidentally discovered that dextrorotatory oxiracetam has an antiepileptic activity and had a significant inhibitory effect on acute epilepsy, especially has a significant inhibitory effect on acute epilepsy seizures. Dextrorotatory oxiracetam has a good inhibitory effect on generalized epilepsy seizure, partial epilepsy seizure and status epilepticus, and dextrorotatory oxiracetam has high bioavailability and low toxicity, as well as suitable for further development as an antiepileptic drug. The invention uses the dextrorotatory oxiracetam having more than 99.0% of purity, effectively excluding the interference from other components. Dextrorotatory oxiracetam is a single active ingredient, making the quality of the drug easier to control, while the effect is clearer. The composition of the dextrorotatory oxiracetam provided by the invention has strong adaptability and can be simply prepared, thereby being advantageous for industrialization.

### Brief description of the drawings

Fig.1 is a diagram of standard curves of oxiracetam.
Fig.2 is a diagram of standard curves of dextrorotatory oxiracetam.
Fig.3 is a bar chart of effects of dextrorotatory oxiracetam on mortality rate and SE occurrence rate of the lithium chloride-pilocarpine epilepsy model rats.
Fig.4 a diagram of effects of the lithium chloride-pilocarpine epilepsy model rats on occurrences of different stages

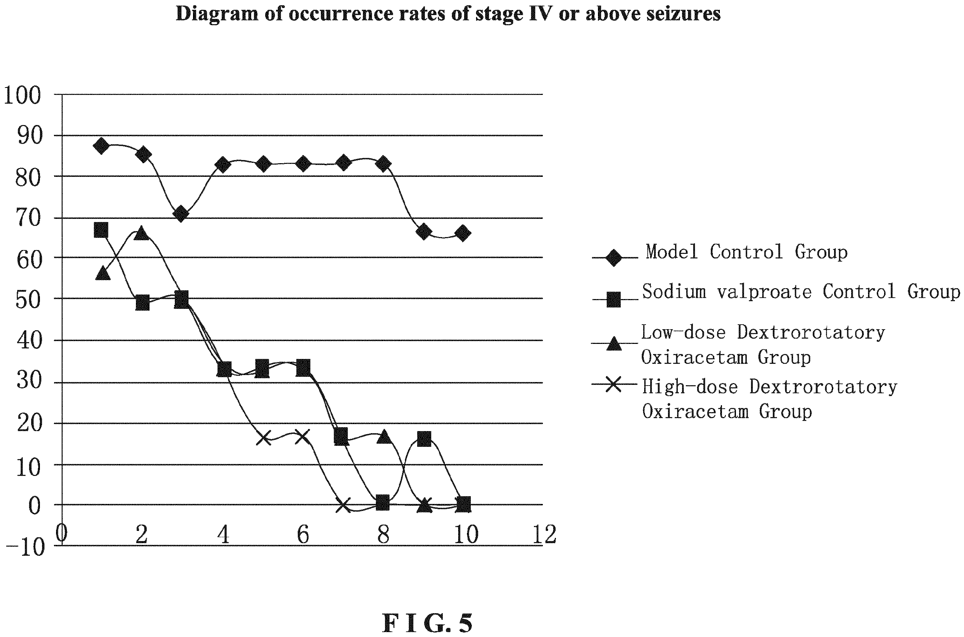

Fig.5 a diagram of occurrence rate of stage IV or above seizures of pentetrazol (PTZ)-ignited rat epilepsy model with dextrorotatory oxiracetam.

### Detailed Description of the embodiments

Hereinafter, several embodiments of the present invention will be described, but the present invention is not limited thereto.

### Example 1

Raw material composition:

| | |
|---|---|
| (a) Dextrorotatory oxiracetam (99.5% of purity) | 200mg/ tablet |
| (b) Lactose | 80mg/ tablet |
| (c) Microcrystalline cellulose | 70mg/ tablet |

For example, to produce 1000 dextrorotatory oxiracetam capsules, the specific preparation method is: Raw materials were passed over 80 mesh sieve. The prescriptive amount of dextrorotatory oxiracetam, lactose, microcrystalline cellulose, are taken and mixed to directly fill into capsules.

### Example 2

Raw material composition:

| | |
|---|---|
| (a) Dextrorotatory oxiracetam (99.6% of purity) | 200mg/ tablet |
| (b) Starch | 34mg/ tablet |
| (c) Microcrystalline cellulose | 60mg/ tablet |
| (d) Talcum powder | 6mg/tablet |
| (e) 2% hydroxypropyl methyl cellulose (K4M model) | adequate amount |

For example, to produce 1000 dextrorotatory oxiracetam tablets, the specific preparation method is: Raw materials were passed over 80 mesh sieve. The prescriptive amount of dextrorotatory oxiracetam, starch, and microcrystalline cellulose were taken and mixed homogenously, then added to soft material made by 2% HPMC solution. After granulating, drying, granulating, the granules were added prescriptive amount of talcum powder and then mixing and tableting.

### Example 3

Raw material composition:

| | |
|---|---|
| (a) Dextrorotatory oxiracetam (99.0% of purity) | 200mg/ tablet |
| (b) Lactose | 80.8mg/ tablet |
| (c) Sodium carboxymethyl starch | 72mg/ tablet |
| (d) Talcum powder | 7.2mg/ tablet |
| (e) 10% polyvinylpyrrolidone | adequate amount |

For example, to produce 1000 dextrorotatory oxiracetam capsules, the specific preparation method is: Raw materials were passed over 80 mesh sieves. The prescriptive amount of dextrorotatory oxiracetam, lactose, sodium carboxymethyl starch were taken and mixed homogenously, then added to soft material made by 10% PVP ethanol solution. After granulating, drying, granulating, the granules were added prescriptive amount of talcum powder and then mixing and filling into capsules.

### Example 4

For example, to produce dextrorotatory oxiracetam injection solution, the specific preparation method is: 50 g of dextrorotatory oxiracetam (99.3% of purity), 150 g of glucose, 500 ml of water for Injection are dissolved in a dilution can, and then mixed under 50∼60°C until totally dissolved. The dissolved solution was cooled to 25 °C. Charcoal was added into the dissolved solution for decolorization. The charcoal was filtered and removed, and then phosphate buffer was added into the dissolved solution to adjust its pH value to 4.0. Then, the water for injection was added to make 5000 ml of the diluted solution. The diluted solution was sealed and sterilized under 105°C for 30 minutes to obtain the dextrorotatory oxiracetam injection solution.

## Claims

1. A dextrorotatory oxiracetam compound or a composition containing dextrorotatory oxiracetam compound for use in preventing or treating epilepsy.

2. A dextrorotatory oxiracetam compound or a composition containing dextrorotatory oxiracetam compound for use in preventing or treating acute epilepsy seizure.

3. A dextrorotatory oxiracetam compound or a composition containing dextrorotatory oxiracetam compound for use in preventing or treating generalized epilepsy seizure.

4. A dextrorotatory oxiracetam compound or a composition containing dextrorotatory oxiracetam compound for use in preventing or treating partial epilepsy seizure.

5. A dextrorotatory oxiracetam compound or a composition containing dextrorotatory oxiracetam compound for use in preventing or treating status epilepticus.

6. The dextrorotatory oxiracetam compound or the composition as claimed in any one of claims 1-5 for use according to any one of claims 1-5, **characterized in that** the dextrorotatory oxiracetam compound or a composition containing the dextrorotatory oxiracetam compound is suitable to be administered at a dose of not less than 400 mg/day.

7. The dextrorotatory oxiracetam compound or the composition as claimed in any one of claims 1-5 for use according to any one of claims 1-5, **characterized in that** the optical purity of the dextrorotatory oxiracetam compound is 99.0% or more, based on weight percent.

8. The dextrorotatory oxiracetam compound or the composition as claimed in any one of claims 1-5 for use according to any one of claims 1-5, **characterized in** containing the dextrorotatory oxiracetam compound, and pharmaceutically acceptable excipients; the optical purity of the composition containing the dextrorotatory oxiracetam compound is 99.0% or more, based on weight percent.

## Patentansprüche

1. Rechtsdrehende Oxiracetamverbindung oder eine Zusammensetzung, enthaltend rechtsdrehende Oxiracetamverbindung, zur Verwendung bei Prävention oder Behandlung von Epilepsie.

2. Rechtsdrehende Oxiracetamverbindung oder eine Zusammensetzung, enthaltend rechtsdrehende Oxiracetamverbindung, zur Verwendung bei Prävention oder Behandlung von akutem Epilepsieanfall.

3. Rechtsdrehende Oxiracetamverbindung oder eine Zusammensetzung, enthaltend rechtsdrehende Oxiracetamverbindung, zur Verwendung bei Prävention oder Behandlung von verallgemeinertem Epilepsieanfall.

4. Rechtsdrehende Oxiracetamverbindung oder eine Zusammensetzung, enthaltend rechtsdrehende Oxiracetamverbindung, zur Verwendung bei Prävention oder Behandlung von partiellem Epilepsieanfall.

5. Rechtsdrehende Oxiracetamverbindung oder eine Zusammensetzung, enthaltend rechtsdrehende Oxiracetamverbindung, zur Verwendung bei Prävention oder Behandlung von Status epilepticus.

6. Rechtsdrehende Oxiracetamverbindung oder Zusammensetzung wie beansprucht in einem der Ansprüche 1-5 zur Verwendung nach einem der Ansprüche 1-5, **gekennzeichnet dadurch, dass** die rechtsdrehende Oxiracetamverbindung oder eine Zusammensetzung, enthaltend rechtsdrehende Oxiracetamverbindung, geeignet ist, in einer Dosis von nicht weniger als 400 mg/Tag verabreicht zu werden.

7. Rechtsdrehende Oxiracetamverbindung oder Zusammensetzung wie beansprucht in einem der Ansprüche 1-5 zur Verwendung nach einem der Ansprüche 1-5, **gekennzeichnet dadurch, dass** die optische Reinheit der rechtsdrehenden Oxiracetamverbindung 99,0 % oder mehr beträgt, basierend auf Gewichtsprozent.

8. Rechtsdrehende Oxiracetamverbindung oder Zusammensetzung wie beansprucht in einem der Ansprüche 1-5 zur Verwendung nach einem der Ansprüche 1-5, **gekennzeichnet durch** Enthalten der rechtsdrehenden Oxiracetamverbindung und pharmazeutisch verträglicher Hilfsstoffe; wobei die optische Reinheit der Zusammensetzung, enthaltend die rechtsdrehende Oxiracetamverbindung, 99,0 % oder mehr beträgt, basierend auf Gewichtsprozent.

## Revendications

1. Un composé d'oxiracétam dextrogyre ou une composition contenant un composé d'oxiracétam dextrogyre pour une utilisation dans la prévention ou le traitement de l'épilepsie.

2. Un composé d'oxiracétam dextrogyre ou une composition contenant un composé d'oxiracétam dextrogyre pour une utilisation dans la prévention ou le traitement de la crise d'épilepsie aiguë.

3. Un composé d'oxiracétam dextrogyre ou une composition contenant un composé d'oxiracétam dextrogyre pour une utilisation dans la prévention ou le traitement de la crise d'épilepsie généralisée.

4. Un composé d'oxiracétam dextrogyre ou une composition contenant un composé d'oxiracétam dextrogyre pour une utilisation dans la prévention ou le traitement de la crise d'épilepsie partielle.

5. Un composé d'oxiracétam dextrogyre ou une composition contenant un composé d'oxiracétam dextrogyre pour une utilisation dans la prévention ou le traitement de l'état épileptique.

6. Le composé d'oxiracétam dextrogyre ou la composition selon l'une quelconque des revendications 1 à 5 pour une utilisation selon l'une quelconque des revendications 1 à 5, **caractérisé en ce que** le composé d'oxiracétam dextrogyre ou une composition contenant le composé d'oxiracétam dextrogyre est approprié pour être administré à une dose non inférieure à 400 mg/jour

7. Le composé d'oxiracétam dextrogyre ou la composition selon l'une quelconque des revendications 1 à 5, pour une utilisation selon l'une quelconque des revendications 1 à 5, **caractérisé en ce que** la pureté optique du composé d'oxiracétam dextrogyre est de 99,0 % ou plus, sur la base du pourcentage en poids

8. Le composé d'oxiracétam dextrogyre ou la composition selon l'une quelconque des revendications 1 à 5 pour une utilisation selon l'une quelconque des revendications 1 à 5, **caractérisé par** le fait de contenir le composé d'oxiracétam dextrogyre, et des excipients pharmaceutiquement acceptables ; la pureté optique de la composition contenant le composé d'oxiracétam dextrogyre est de 99,0% ou plus, sur la base du pourcentage en poids.
